# EUROPEAN PATENT APPLICATION

(11) **EP 0 914 822 A1**
(43) Date of publication of application: **12.05.1999**
(21) Application number: 97907281.6
(22) Date of filing: 12.03.1997
(51) Int. Cl.: A61K 31/135, A61K 9/14, A61K 9/20, A61K 47/38

(54) **RAPID-RELEASE MICRODISPERSIBLE ECADOTRIL PREPARATION**

(30) Priority: 14.03.1996 JP 58113/96
(71) Applicant: Bayer Aktiengesellschaft, 51368 Leverkusen (DE)
(72) Inventor: TSUKADA, Takayuki, Itami-shi, Hyogo 664 (JP); SUZUKI, Yusuke, Izumi-shi, Osaka 594 (JP); OGURA, Toshihiro, Suita-shi, Osaka 565 (JP)
(74) Representative: Bader, Axel Jochen
(86) International application number: JP9700773
(87) International publication number: WO9733571

(57) **Abstract**

A process for producing a rapid-release microdispersible ecadotril preparation, which includes the step of treating a mixture of ecadotril and a disintegrator with a wettability improver, or the step of combining ecadotril treated with a wettability improver and a disintegrator.

## Description

### Field of the Invention

The present invention relates to a method for producing a preparation containing Ecadotril, and in particular, to a method for producing a rapid-release microdispersible preparation which rapidly disintegrates and allows the drug particles to be rapidly microdispersed. The present invention also relates to a rapid-release microdispersible preparation containing Ecadotril, wherein the preparation is obtained by the producing method of the present invention, and a rapid-release microdispersible tablet formulated from this preparation.

### Background of the Invention

Ecadotril (N-[(S)-α-(mercaptomethyl)hydrocinnamoyl]glycine, benzyl ester, acetate(ester); N-[(S)-α-(acetylthiomethyl)hydrocinnamoyl]glycine, benzyl ester) is an enkephalinase inhibitor used as a hypotensive drug, a cardiac insufficiency therapeutic drug, or the like. Ecadotril is insoluble in water and has a low melting point and high plasticity (solubility in water: about 33 µg/ml (37°C), melting point: 70 to 74°C). Therefore, when formulated into tablets, capsules, or the like by conventional methods, sufficient absorbability of Ecadotril in the digestive tract cannot be obtained because of low dispersibility of drug particles. Thus, a preparation of Ecadotril with improved bioavailability, in particular a tablet with high content, has been demanded. Generally, it is known that in order to obtain sufficient absorbability of such water-insoluble drugs, the solubility (dissolving rate) of the drugs is enhanced. For example, Japanese Laid-Open Patent Publication No. 7-76516 discloses a method for spray drying dispersions in which finely ground drug particles and a hydrophilic excipient are dispersed in water. However, according to this method, when powder to be obtained is formulated into tablets, there is a possibility that drug particles come into close contact with each other thereby decreasing the disintegration property and absorbability of the preparation. Furthermore, in the case where water-insoluble drugs such as Ecadotril with a low melting point and high plasticity are used, particles are likely to aggregate. Therefore, such drugs are not suitable for being formulated into tablets. Thus, there has been no techniques to formulate Ecadotril into a rapid-release microdispersible preparation efficiently, in particular a tablet with high content.

### Summary of the Invention

The objective of the present invention is to provide a method for producing a preparation containing Ecadotril, wherein the preparation is rapidly absorbed by digestive tract. More specifically, the objective of the present invention is to provide a method for producing a preparation (a rapid-release microdispersible preparation) which disintegrates and allows Ecadotril to be rapidly and finely dispersed into primary particles.

According to the present invention, the method for producing a rapid-release microdispersible preparation containing Ecadotril comprises the step of treating a mixture containing Ecadotril and a disintegrator with a wettability improver, or the step of combining Ecadotril treated with a wettability improver and a disintegrator.

In a preferred embodiment, the method of the present invention comprises the steps of preparing an aqueous suspension by wet grinding a mixture containing Ecadotril and a wettability improver, and performing fluidized-bed granulation in which powder containing a disintegrator is sprayed with the aqueous suspension.

In another preferred embodiment, the method of the present invention comprises the steps of dispersing a disintegrator in an aqueous suspension obtained by wet grinding a mixture containing Ecadotril and a wettability improver to obtain a spray suspension, and performing Spray-Drying in which the spray suspension is spray dried.

In still another preferred embodiment, the method of the present invention comprises the steps of spray drying an aqueous suspension obtained by wet grinding a mixture containing Ecadotril and a wettability improver to obtain fine granules, and adding a disintegrator to the fine granules and mixing the disintegrator with the fine granules.

In another preferred embodiment, the method of the present invention comprises the steps of wet grinding a mixture containing Ecadotril and a wettability improver to obtain an aqueous suspension, performing coating granulation in which core particles are sprayed with the aqueous suspension, and combining a disintegrator and the particles obtained from the step of coating granulation.

In a preferred embodiment, the aforementioned wettability improver is a water-soluble polymer or a surfactant. Preferably, the wettability improver is hydroxypropylcellulose. Preferably, the content of the wettability improver in the rapid-release microdispersible preparation is 0.5 to 8 parts by weight based on 100 parts by weight of Ecadotril.

In a preferred embodiment, the aforementioned disintegrator is sodium crosscarmellose. Preferably, the content of the disintegrator in the rapid-release microdispersible preparation is 5 to 40 parts by weight based on 100 parts by weight of Ecadotril.

In a preferred embodiment, the content of Ecadotril in the rapid-release microdispersible preparation is about 60% by weight or more, preferably about 65 to 85% by weight based on the total weight of the preparation.

According to the present invention, there is also provided a rapid-release microdispersible preparation containing Ecadotril, wherein the preparation is produced by the method of the present invention.

In a preferred embodiment, the rapid-release microdispersible preparation of the present invention includes a disintegration portion.

According to the present invention, there is also provided a rapid-release microdispersible tablet containing Ecadotril, wherein the tablet is obtained by tableting the rapid-release microdispersible preparation of the present invention.

In a preferred embodiment, the tablet of the present invention is obtained by the method comprising the steps of dispersing about 15 to 25 parts by weight of disintegrator in an aqueous suspension containing 100 parts by weight of Ecadotril and about 3 to 5 parts by weight of water-soluble polymer to obtain a spray suspension, spray drying the spray suspension to obtain fine granules, and tableting the fine granules. The content of Ecadotril in the tablet is about 60% by weight or more.

In another preferred embodiment, the tablet of the present invention is obtained by the method comprising the steps of dispersing about 19 to 21 parts by weight of sodium crosscarmellose in an aqueous suspension containing 100 parts by weight of Ecadotril and about 3 to 5 parts by weight of hydroxypropylcellulose to obtain a spray suspension, spray drying the spray suspension to obtain fine granules, and tableting the fine granules. The content of Ecadotril in the tablet is about 80% by weight.

In another preferred embodiment, the tablet of the present invention is obtained by the method comprising the step of dispersing about 19 to 21 parts by weight of sodium crosscarmellose in an aqueous suspension containing 100 parts by weight of Ecadotril with a 50% average particle size of about 10 µm or less and about 3 to 5 parts by weight of hydroxypropylcellulose to obtain a spray suspension, spray drying the spray suspension to obtain fine granules, and tableting the fine granules under a compression pressure of about 0.3 to 0.6 tons. The content of Ecadotril in the tablet is about 80% by weight.

### Brief Description of the Drawings

Figure 1 shows curves representing dissolution of preparations in Examples according to the present invention and Comparative Examples.
Figure 2 shows curves representing dissolution of preparations of Examples according to the present invention.
Figure 3 shows curves representing absorption of preparations of Examples according to the present invention and Comparative Examples.

### Detailed Description of the Invention

According to the present invention, a rapid-release microdispersible preparation containing Ecadotril is prepared using Ecadotril, a wettability improver and a disintegrator. The rapid-release microdispersible preparation of the present invention has a form in which Ecadotril particles with their wettability improved by the wettability improver are mixed with the disintegrator in an aggregated form.

Each component used for producing the rapid-release microdispersible preparation of the present invention will be described.

### (Ecadotril)

It is advantageous that the particle size of Ecadotril used in the present invention is as small as possible, because the dissolving rate of Ecadotril increases as the particle size thereof gets smaller. Thus, the 50% average particle size, for example, is adjusted to 10 µm or less, preferably 3 µm or less. In the case where fine drug particles are not obtained using a crystallization process, the drug is preferably ground by a conventional grinder so as to increase the substantial surface area of the drug, thereby enhancing the dissolving rate of the drug. As a grinder, dry grinders such as a jet mill, a ball mill, and a hammer mill may be used; and wet grinders such as a pressure-type homogenizer, a colloid mill, a nanomizer, and a roller mill may be used. Ecadotril alone or mixtures thereof with additives can be ground by these grinders.

The content of Ecadotril is 25 to 85% by weight, preferably 35 to 85% by weight, especially preferably 60% by weight or more, most preferably 65 to 85% by weight based on the total weight of the preparation obtained by the method of the present invention. When the content of Ecadotril is less than 25% by weight, although fine-dispersibility of a preparation to be obtained increases, the preparation to be obtained becomes bulky and difficult to be administered, in the case when a predetermined amount (e.g., 50 mg) or more of Ecadotril is contained in a preparation. When the content exceeds 85% by weight, there is a possibility that the desired fine-dispersibility of the preparation cannot be obtained, in the case when a preparation to be obtained is tableted. However, it is clinically advantageous, when tableted, that the higher the content of Ecadotril in a single tablet, the fewer the number of tablets administrated in one dosage.

### (Wettability Improver)

The above-mentioned wettability improver is used for the purpose of improving the wettability of particle surfaces of pulverized Ecadotril so as to enhance the dissolving rate thereof. More specifically, when the wettability of Ecadotril is improved due to a thin layer of the wettability improver formed on the surfaces of Ecadotril crystals, the water repellency of Ecadotril is decreased and therefore it is likely to be dispersed in water. Examples of the wettability improver include water-soluble polymers such as hydroxypropylcellulose (HPC), hydroxypropylmethylcellulose (HPMC), methylcellulose, polyvinyl pyrrolidone, polyvinyl alcohol, and gelatin; and surfactants such as sodium lauryl sulfate, polyethyleneglycol monostearate, glycerin monostearate, polyoxyethylenesorbitan aliphatic acid ester, polyoxyethylene hardened castor oil, and polyoxyethylenepolyoxypropylene glycol. Among them, hydroxypropylcellulose and hydroxypropylmethylcellulose are preferable. Hydroxypropylcellulose is most preferable. The content of the wettability improver is 0.5 to 8 parts by weight, preferably 2 to 6 parts by weight, more preferably 3 to 5 parts by weight based on 100 parts by weight of Ecadotril.

### (Disintegrator)

The disintegrator is an additive imparting a disintegration property to the preparation and is used for rapidly dispersing Ecadotril. Although being insoluble in water, the disintegrator has excellent affinity with water. The disintegrator swells upon contact with water to disintegrate the preparation, thereby accelerating the contact between Ecadotril and water. Examples of the disintegrator include calcium carmellose (CMC-Ca), low-substituted hydroxypropylcellulose, partially α-starch, sodium carboxymethyl starch, low-substituted sodium carboxymethyl starch, sodium crosscarmellose, crystalline cellulose, and crosspovidone. Among them, calcium carmellose, low-substituted hydroxypropylcellulose, sodium crosscarmellose, and crosspovidone are preferable. Sodium crosscarmellose is most preferable. The content of the disintegrator is preferably 5 to 40 parts by weight, more preferably 10 to 25 parts by weight based on 100 parts by weight of Ecadotril.

### (Other Additives)

In addition to the above-mentioned wettability improver and disintegrator, the rapid-release microdispersible preparation of the present invention can, if desired, contain one or more pharmaceutically acceptable additives such as an excipient, a binder, a lubricant, an antistatic agent, a colorant, a flavoring agent, and a stabilizer as another additive.

The excipient is used for imparting a predetermined size and weight to the preparation. The excipient used in the present invention is preferably a water-soluble excipient, or a mixture of a water-soluble excipient and a hydrophilic excipient. Because of the excellent affinity with and solubility in water, the water-soluble excipient weakens the binding amongst Ecadotril particles in an aqueous solvent and accelerates the disintegration of the preparation. Furthermore, the water-soluble excipient is preferably pulverized in order to help dilute the drug. Examples of the water-soluble excipient include lactose, sucrose, and mannitol; in particular, lactose is preferable. The hydrophilic excipient has excellent affinity with water although being insoluble in water. Therefore, the hydrophilic excipient intervenes in the contact between the preparation particles and water so as to swell the preparation particles. This improves the disintegration property and dispersibility of the preparation. Examples of the hydrophilic excipient include starches such as corn starch, potato starch, and hydroxypropyl starch; and inorganic salts such as silicic anhydride and anhydrous calcium hydrogenphosphate. In particular, corn starch is preferable. With a proviso, when desired to obtain a preparation with high Ecadotril content, Ecadotril may be formulated without any excipients by, preferably, employing the Spray-Drying method of the present invention as described below in detail.

The above-mentioned binder is used for imparting a binding force to a mixture of powder component so as to prepare stable granules. The binder suppresses pulverization of a granulated substance obtained by wet granulation, so that the strength of granules can be maintained. Examples of the binder include methylcellulose, polyvinyl pyrrolidone, hydroxypropylcellulose, hydroxypropylmethylcellolose, soluble α-starch, polyvinyl alcohol, gelatin, and dextrine. Among them, hydroxypropylcellulose and hydroxypropylmethylcellulose are preferable because they also improve wettability.

The above-mentioned lubricant is used for preventing or solving various problems, such as sticking, binding, and capping, caused by the adhesion between the substance to be tableted and the pestle and mortar due to compression. It is also used for helping to supply the granulated substance smoothly, thereby preventing the unevenness of weight of tablets or capsules. Examples of the lubricant include magnesium stearate (St-Mg), talc, sucrose aliphatic acid ester, and simethicone.

As the above-mentioned antistatic agent, light silicic anhydride can be used. As the above-mentioned colorant, iron oxide, lake pigment, and the like can be used. As the above-mentioned flavoring agent, various perfumes can be used. As the above-mentioned stabilizer, sodium hydrogensulfite can be used.

The content of the above-mentioned additives can be appropriately adjusted, respectively, depending upon the use, dosage form to be selected and its size, the production method, and the kind and amount of the other additives.

### (Producing Method of the Rapid-release Microdispersible Preparation)

The method for producing a rapid-release microdispersible preparation containing Ecadotril of the present invention comprises the following step (a) or (b):
(a) a step of treating a mixture containing Ecadotril and a disintegrator with a wettability improver; or
(b) a step of combining Ecadotril, which has been treated with a wettability improver, and a disintegrator.

Each step will be described.

### Step (a)

In this step, Ecadotril is mixed with a disintegrator, and the mixture is treated with a wettability improver. The treatment of the mixture with the wettability improver may be conducted by granulating the mixture while being in contact with the wettability improver, preferably, in the presence of water. Thus, the wettability of Ecadotril may be improved in the course of granulation. As the granulation method employed in the step (a), a kneading method, a stirring granulation method, a fluidized-bed granulation method and the like, as described later, can be used.

During this granulation process, if desired, a binder can be used. However, it is preferable to use a wettability improver, which also acts as a binder, such as hydroxypropylcellulose and hydroxypropylmethylcellulose, because this can save the use of a binder.

### Step (b)

In this step, Ecadotril is treated with a wettability improver, and then Ecadotril treated with the wettability improver is combined with a disintegrator. The treatment of Ecadotril with the wettability improver may be conducted by contacting Ecadotril with the wettability improver, preferably, in the presence of water. Thereby, Ecadotril particles with their surfaces covered with coatings of the wettability improver are obtained. Preferably, the treatment is conducted by wet grinding a mixture containing Ecadotril and a wettability improver to prepare an aqueous suspension. Then, the Ecadotril particles treated with the wettability improver and a disintegrator are combined. Preferably, the mixture containing the Ecadotril particles and the disintegrator is subjected to granulation. As a result, Ecadotril treated with the wettability improver is integrated with the disintegrator to give a preparation of the present invention. As the granulation method employed in the step (b), a kneading method, a stirring granulation method, a fluidized-bed granulation method, a coating granulation method, a Spray-Drying method, and the like are preferable.

During this granulation process, if desired, a binder can be used. However, it is preferable to use a wettability improver, which also acts as a binder, such as hydroxypropylcellulose and hydroxypropylmethylcellulose, because this can save the use of a binder.

Hereinafter, examples of the granulation method will be shown.

### (1) Kneading method

An aqueous solution of a wettability improver is added to mixed powder containing Ecadotril, a disintegrator and, if desired, any other additives (excipient, binder, etc.). The resultant mixture is wet-kneaded by using a kneader. The aqueous solution of the wettability improver can further contain a binder. Consequently, the surface of Ecadotril crystals is treated with the wettability improver. As the kneader, a ribbon blender, a single screw kneader or a double screw kneader, and the like are used. The kneaded material thus obtained is granulated by screening, an extrusion granulation method, or the like. The granules are dried and sieved to give the preparation of the present invention. In the above-mentioned method, a powdery wettability improver or a liquid wettability improver itself can be added to the mixture containing Ecadotril, a disintegrator and, if desired, any other additives, in place of the aqueous solution of the wettability improver. Then, a binder solution or water can be added to the mixture, and the resultant mixture can be wet-kneaded and granulated as described above to give the preparation of the present invention.

Alternatively, an aqueous solution of the wettability improver can be added to Ecadotril in an appropriate amount, and the resultant mixture can be finely ground by a wet grinding method to prepare an aqueous suspension. A disintegrator and, if desired, any other additives can be mixed with the aqueous suspension, and the resultant mixture can be wet-kneaded and granulated as described above to give the preparation of the present invention. The aqueous suspension can further contain an excipient, preferably a water-soluble excipient.

### (2) Stirring granulation method

This is a method for obtaining the preparation of the present invention by stirring granulation, drying and sieving in the same manner as the kneading method of the above item (1), using a blade rotary mixer or a low share mixer (granulation at a high rotation speed for a short period of time is possible). Compared with the kneading method, this method is less effective in putting the wettability improver onto the crystal surfaces of Ecadotril. However, such effectiveness can be improved by adding an appropriate amount of an organic solvent such as ethanol. Alternatively, in the same way as in the item (1), a wet-ground aqueous suspension containing Ecadotril and a wettability improver (and an excipient, if desired) is added to a disintegrator or mixed powder of a disintegrator and an arbitrary additive. Then, the mixture is subjected to stirring granulation.

### (3) Fluidized-bed granulation method

An aqueous solution of a wettability improver is added to mixed powder containing Ecadotril, a disintegrator and, if desired, any other additives (excipient, binder, etc.). The mixture is granulated by using a fluidized-bed granulator to give the preparation of the present invention. The aqueous solution of the wettability improver can further contain a binder. Alternatively, a wet-ground aqueous suspension containing Ecadotril and a wettability improver (and an excipient, if desired) is prepared, and then a fluidized disintegrator and, preferably, an excipient are granulated while being sprayed with the wet-ground aqueous suspension to give the preparation of the present invention. As a spray method, either of a top spray, a side spray or a tangential spray can be used.

### (4) Coating granulation method

This is a method for coating granulation through spraying a wet-ground aqueous suspension containing Ecadotril and a wettability improver (and an excipient, if desired) onto core particles by using a fluidized-bed granulator, a pivoted fluidized-bed granulator, or a WURSTER-type granulator. The core particles can be any particles having a size of about several 10 µm to 300 µm. Examples of the core particles preferably include D-mannitol crystal, NONPAREL, crystalline cellulose particles, granulated sugar and spray-dried lactose. The particles containing Ecadotril and the wettability improver obtained by the coating granulation method may be then combined with a disintegrator by any means.

### (5) Spray-Drying method

Preferably, as shown in the following example 8, a mixture containing Ecadotril and a wettability improver is wet ground to produce an aqueous suspension, and then a disintegrator is dispersed in the suspension to prepare a spray suspension. The spray suspension is spray dried by using a spray dryer to give a preparation of the present invention. According to this method, the disintegrator becomes sufficiently swelled in the spray suspension. As a result, the efficiency for preventing adhesion of the active ingredients with each other upon tableting granules obtained by spray drying becomes higher even in the absence of an excipient, whereby a tablet being more rapid-release microdispersible is obtained. Alternatively, the preparation of the present invention can be obtained by spray drying a wet ground aqueous suspension containing Ecadotril, a wettability improver and a disintegrator (and an excipient, if desired). Alternatively, an aqueous suspension obtained by wet grinding a mixture containing Ecadotril and a wettability improver is spray dried to give fine granules, and then a disintegrator is added to and mixed with the fine granules, thereby a preparation of the present invention can be obtained.

According to the Spray-Drying method, the surface of Ecadotril can be well coated with a wettability improver since the wettability improver and Ecadotril can be efficiently contacted in water. Thus, Ecadotril can be stably dispersed in a suspension. As a result, the suspension can be dried while Ecadotril is homogeneously dispersed. Further, a preparation with a high Ecadotril content (e.x., about 80% by weight content) can be prepared without using excipients.

The concentration of Ecadotril in a spray suspension is normally about 10 to 35% by weight, preferably about 15 to 25% by weight. If the concentration is very low, the productivity will be reduced since the amount of the suspension to be treated is increased. Whereas, if the concentration is very high, a preferable suspension state is not obtained.

A wettability improver used in a spray suspension is preferably a water-soluble polymer, especially preferably hydroxypropylcellulose. A disintegrator used in a spray suspension is preferably sodium crosscarmellose. A preferable spray suspension can be obtained by dispersing about 15 to 25 parts by weight, more preferably about 19 to 21 parts by weight of disintegrator into an aqueous suspension containing 100 parts by weight of Ecadotril and about 3 to 5 parts by weight of a water-soluble polymer.

As a condition for spray drying, it is required to set a condition that the temperature of Ecadotril is not equal to or beyond its melting point (about 72 °C). Normally, a supply rate of a suspension may be adjusted so as to allow the temperature of a spray dryer at the outlet to be about 50 to 60 °C. As a spray dryer, for example, Oogawara-kakoki L-8 type spray dryer or the like can be used.

### (Rapid-Release Microdispersible Preparation)

The rapid-release microdispersible preparation of the present invention obtained by the method as described above may be in a form of granules or powders. In the present specification, the terms "granules " and "powders" are used in a common meaning used in the art and are, in particular, defined in accordance with The 12th revised Japanese Pharmacopoeia. In the present specification, the term "powders" includes fine granules.

According to the present invention, tablets and capsules prepared by using the rapid-release microdispersible preparation of the present invention in a form of granules or powders are also provided. Tablets can be obtained by compressing the preparation of the present invention, if desired, together with optional additives. Capsules can be obtained by encapsulating the preparation of the present invention, if desired, together with optional additives. As is the case with the preparation of the present invention in a form of granules or powders, these tablets and capsules are also rapid-release microdispersible preparations of Ecadotril.

When the preparation of the present invention is further formulated into other dosage forms such as tablets and capsules, disintegration portions can be provided so as to enhance its disintegration. The disintegration portions contain a disintegrator. This disintegrator can be similar to that contained in the preparation of the present invention. The disintegrator is contained in the disintegration portions in an amount of 2 to 100% by weight, preferably 5 to 10% by weight.

The disintegration portions can further contain at least one pharmaceutically acceptable additive, such as an excipient, a binder, a lubricant, an antistatic agent, a colorant, a flavoring agent and a stabilizer. These additives can be similar to those used in the preparation of the present invention.

The content of the additive contained in the disintegration portions can be appropriately adjusted, depending upon the purpose of use, dosage form to be selected and its size, the production method, and the kind and amount of the other additives.

The disintegration portions can be produced by various arbitrary granulation methods substantially the same as those used for the preparation of the present invention, except that Ecadotril and a wettability improver are not used. Alternatively, a dry granulated and shredded mixed powder containing a disintegrator and an arbitrary additive or mixed powder itself can be used for the disintegration portions.

In the case where the disintegration portions are provided, the mixing ratio (by weight) of the preparation of the present invention to the disintegration portion is preferably 1:1 to 1:0.05.

Capsules of the preparation of the present invention can be obtained by filling capsules with the preparation of the present invention and, if required, disintegration portions, using an ordinary encapsulation machine. Tablets of the preparation of the present invention can be generally obtained by mixing a lubricant in an amount of 0.1 to 2% by weight with the preparation of the present invention or a mixture containing the preparation of the present invention and the disintegration portions, and tableting the resultant mixture, using an ordinary tableting machine. The tableting can be conducted at a relative low tableting pressure such as normally about 0.3 to 0.6 tons. As the lubricant, magnesium stearate (St-Mg), talc, sucrose aliphatic acid ester, simethicone, etc. can be used as described above. Regarding the tablets, if required, they are further coated with a coating material.

The preparation of the present invention, or the rapid-release microdispersible tablets or capsules made of the preparation rapidly disintegrate into microparticles with a size of about 1 to 30 µm in water or gastric juice without forming a larger primary disintegrated fragments, due to the presence of the disintegrator and the wettability improver on the Ecadotril surface. Moreover, they do not form a hydrophobic associated complex of Ecadotril. Namely, Ecadotril rapidly disintegrates and is dispersed into the original primary particles. Dispersed Ecadotril mainly dissolves at the area from the duodenum to the upper portion of small intestine, where bile is abundant, and is thus rapidly absorbed in a body. Furthermore, the disintegration portions enable the disintegration time of the preparation to be remarkably shortened. Particularly, when the preparation is formulated into tablets without disintegration portions, Ecadotril particles may be pressed against each other by a compression pressure in the course of tableting. As a result, the disintegration of the preparation is likely to become slow. Therefore, the presence of the disintegration portions is preferable.

### Examples

Hereinafter, the present invention will be specifically described by way of illustrative examples. It should be noted that the present invention is not limited thereto.

### (Example 1)

Ecadotril powder is ground by a jet mill to obtain fine-ground powder with a 50% average particle size of about 5 µm. Then, 120.0 g of the Ecadotril fine-ground powder, 66.0 g of lactose ground powder, 28.8 g of corn starch, and 18.0 g of calcium carmellose (CMC-Ca) are mixed with each other. Separately, 5.2 g of hydroxypropylcellulose (HPC-SL) is dissolved in 94.8 g of water to prepare a 5.2% wettability improver solution. Then, 88.46 g of the wettability improver solution is added to 223.1 g of the mixed powder and the resultant mixture is kneaded by using a double screw kneader. This kneaded material is granulated, dried, and sized by a conventional method, thereby preparing sized granules (which are sized in an appropriate particle size distribution). Then, 19.8 g of the sized granules are mixed with 0.20 g of magnesium stearate (St-Mg) to obtain granules for tableting (which are lubricated granules). Then, 200 mg of the lubricated granules are compressed by a static compressor under a pressure of 0.45 tons, whereby tablets each having a diameter of 8.0 mm are obtained.

### (Example 2)

First, 2500.0 g of 2.4% aqueous solution of hydroxypropylcellulose (HPC-SL) is added to 1000.0 g of Ecadotril to prepare Ecadotril dispersions. The Ecadotril dispersions are ground by a wet grinder (sand mill) so as to prepare a suspension in which 50% average particle size of Ecadotril is about 6 µm. Then, 300 g of water is added to 700 g of the Ecadotril suspension to prepare a spray suspension. Separately, 302.0 g of lactose, 40.0 g of corn starch, and 40.0 g of calcium carmellose (CMC-Ca) are mixed with each other to prepare mixed powder. Then, 382.0 g of the mixed powder is sprayed with 1000 g of the spray suspension in a fluidized-bed granulator (top spray), and dried, whereby granules are prepared. Next, 17.82 g of the granules are mixed with 0.18 g of magnesium stearate (St-Mg) to prepare lubricated granules. Then 300 mg of the lubricated granules are tableted by a static compressor under a pressure of 0.57 tons, whereby tablets each having a diameter of 9.0 mm are obtained.

### (Example 3)

First, 100.0 g of D-mannitol is dissolved in 700 g of Ecadotril suspension containing HPC-SL as prepared in Example 2, and 200 g of water is added to the suspension to prepare a spray suspension. Separately, 242.0 g of spray dry lactose (spherical particles with a 50% average particle size of 70 µm) is mixed with 40.0 g of calcium carmellose (CMC-Ca) to prepare mixed powder. Then, 1000 g of the spray suspension are sprayed onto 282.0 g of the mixed powder in a WURSTER-type fluidized-bed granulator (bottom spray), whereby granules are prepared. Next, 17.82 g of the granules are mixed with 0.18 g of magnesium stearate (St-Mg) to prepare lubricated granules. Then, 300 mg of the lubricated granules is tableted by a static compressor under a pressure of 0.57 tons, whereby tablets each having a diameter of 9.0 mm are obtained.

### (Example 4)

First, 1000 g of water is added to 3500 g of the Ecadotril suspension containing HPC-SL as prepared in Example 2, and 500.0 g of D-mannitol is dissolved in the resultant suspension to prepare a spray suspension. Separately, 1210 g of spray dry lactose is mixed with 200.0 g of calcium carmellose (CMC-Ca) to prepare mixed powder. Then, 5000 g of the spray suspension are sprayed onto 1410 g of the mixed powder in a pivoted fluidized-bed granulator (tangential spray) to prepare granules. Next, 17.82 g of the granules are mixed with 0.18 g of magnesium stearate (St-Mg) to prepare lubricated granules. Then, 300 mg of the lubricated granules are tableted by a static compressor under a pressure of 0.57 tons, whereby tablets each having a diameter of 9.0 mm are obtained.

### (Example 5)

First, 900.0 g of 1.33% aqueous solution of hydroxypropylcellulose (HPC-SL) is added to 300.0 g of Ecadotril to prepare Ecadotril dispersions. The Ecadotril dispersions are ground by a wet grinder (sand mill) so as to prepare a suspension in which 50% average particle size of Ecadotril is about 6 µm. Following 50.5 g of water is added to 400.0 g of the suspension, 29.5 g of D-mannitol is dissolved in the suspension to prepare spray suspension. The suspension is spray dried with a spray dryer, whereby fine granules are prepared. One point five g of sodium crosscarmellose (Asahi Chemical Industry Co., Ltd. : Ac-Di-Sol) and 0.15 g of magnesium stearate (St-Mg) are added to 13.35 g of the fine granules and the mixture is mixed to prepare lubricated fine granules. Then, 150 mg of the lubricated fine granules are tableted by a static compressor under a pressure of 0.40 tons, whereby tablets each having a diameter of 7.5 mm are obtained.

### (Example 6)

First, 20.9 g of lactose, 30.25 g of crystalline cellulose (MCC), and 2.75 g of calcium carmellose (CMC-Ca) are mixed with each other to prepare mixed powder (disintegration portions). Then, 49.0 g of the mixed powder is mixed with 198.0 g of the sized granules prepared in Example 1. Next, 3.0 g of magnesium stearate (St-Mg) is mixed with the mixture to prepare lubricated granules. Then, 250 mg of the lubricated granules are tableted by a static compressor under a pressure of 0.5 tons, whereby tablets each having a diameter of 8.5 mm are obtained.

### (Example 7)

First, 20.9 g of lactose, 30.25 g of crystalline cellulose (MCC), 2.75 g of calcium carmellose (CMC-Ca), and 1.0 g magnesium stearate (St-Mg) are mixed with each other to prepare mixed powder. The mixed powder is tableted by a conventional dry method, and the tablets thus obtained are shredded to prepare dry granules (disintegration portions). Then, 50.0 g of the dry granules are mixed with 198.0 g of the sized granules prepared in Example 1. Next, 2.0 g of magnesium stearate (St-Mg) is mixed with the mixture to prepare lubricated granules. Then, 250 mg of the lubricated granules are tableted by a static compressor under a pressure of 0.51 tons, whereby tablets each having a diameter of 8.5 mm are obtained.

### (Example 8)

First, 100.2 g of water is added to 400.0 g of the Ecadotril suspension containing HPC-SL as prepared in Example 5, and 19.8 g of sodium carmellose is dispersed in the resultant suspension to prepare a spray suspension. The suspension is spray dried with a spray dryer, whereby fine granules are prepared. Zero point one two g of magnesium stearate (St-Mg) is added to 12.38 g of the fine granules and the mixture is mixed to prepare lubricated fine granules. Then, 125 mg of the lubricated fine granules are tableted by a static compressor under a pressure of 0.35 tons, whereby tablets each having a diameter of 7.0 mm are obtained.

### (Comparative Example 1)

Ecadotril powder is ground by a jet mill to obtain fine-ground powder with a 50% average particle size of about 5 µm. Then, 10.0 g of the Ecadotril fine-ground powder, 5.4 g of lactose, 1.5 g of corn starch, 1.5 g of crystalline cellulose (MCC), 1.0 g of sodium crosscarmellose (Ac-Di-Sol; Asahi Chemical Industry Co., Ltd.), 0.1 g of CARPLEX 67 (silicon dioxide hydrate; Shionogi & Co., Ltd.) and 0.25 g of magnesium stearate (St-Mg) are mixed with each other. The mixed powder is formulated into slugs by a conventional dry method, and the slugs are shredded to prepare dry granules. Next, 10.0 g of the dry granules are mixed with 0.125 g of magnesium stearate (St-Mg) to prepare lubricated granules. Then, 200 mg of the lubricated granules are tableted by a static compressor under a pressure of 0.45 tons, whereby tablets each having a diameter of 8.0 mm are obtained.

### (Comparative Example 2)

First, 10.0 g of the Ecadotril fine-ground powder prepared in Comparative Example 1, 4.1 g of lactose, 7.5 g of STARCH 1500 (partially α-starch (KARACON)), 0.2 g of AEROSIL 130 (light silicic anhydride; Nippon Aerosil), and 0.70 g of magnesium stearate (St-Mg) are mixed with each other. Then, 225 mg of the mixed powder is filled into No. 2 hard capsules to obtain capsules.

The compositions of the preparations obtained in Examples 1 to 8 and Comparative Examples 1 and 2 are shown in Table 1.

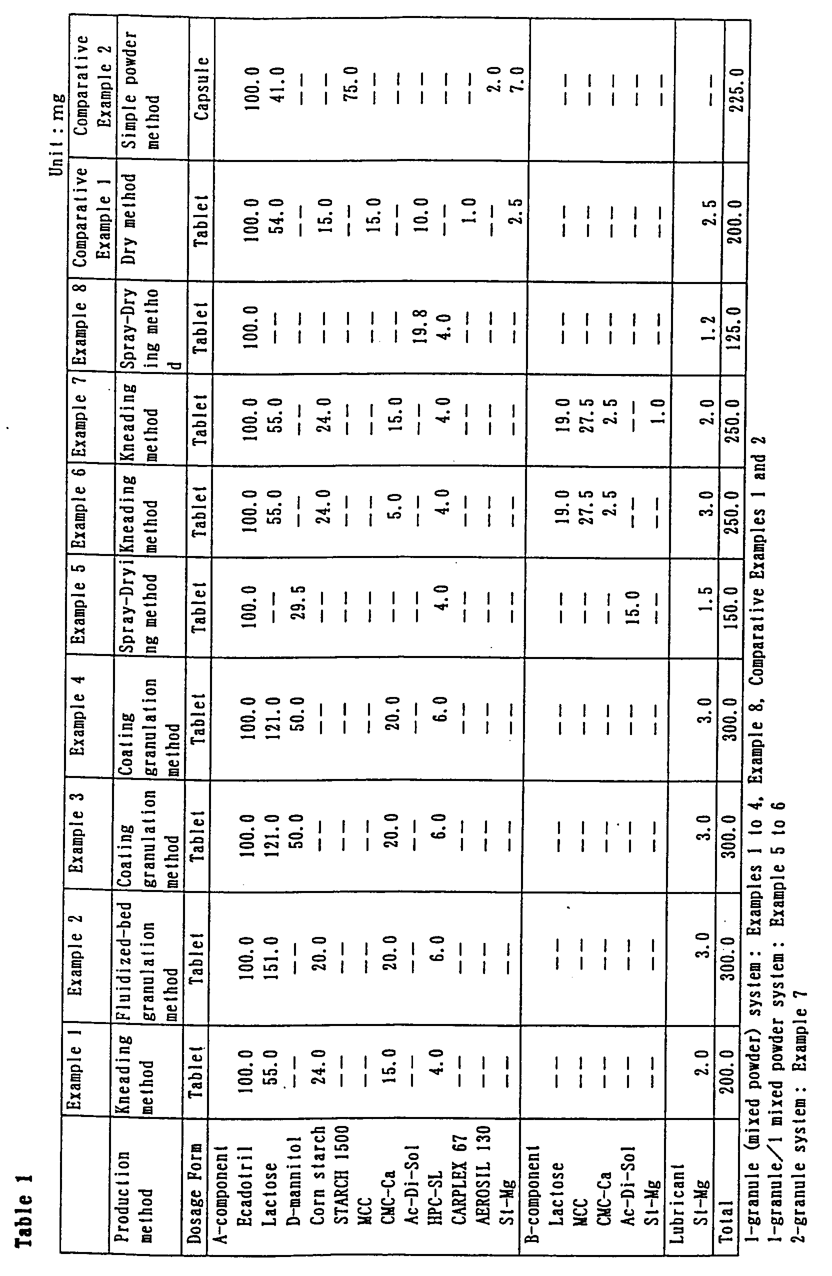

Regarding the above-mentioned preparations, tests were conducted on disintegration, dissolution and absorbability under the following conditions.

### (1) Disintegration test: conducted according to Methods in accordance with Japanese Pharmacopoeia

### (2) Dissolution test (in vitro test)

Apparatus: Dissolution test machine in accordance with The 12th revised Japanese Pharmacopoeia using the paddle method at a stirring speed 100 rpm.
Test solution: Distilled water 900 ml, 37°C
Collection of solution: Eluates are collected 5, 10, 15, 30, 45, and 60 minutes after the commencement of dissolution, and the respective eluates are filtrated through EKICHRODISK 13 (HLC-DISK13; Kanto Chemical Co., Ltd.) aqueous filter (0.22 µm). Then, about 1.5 ml each of the filtrates is collected. One milliliter of this filtrate or standard solution is mixed with 1 ml of internal standard, and 25 µl of the mixed solution is injected to HPLC.

The Internal standard is 40 µg/ml of Flurbiprofene solution in 75% acetonitrile

The solubility of Ecadotril in water is 0.033 mg/ml (37°C), and the maximum dissolution amount in the present test is about 30% in the case of 100 mg of the preparation.

### (3) Absorption test (in vivo test)

One hundred milligrams of the preparation was administered together with 100 ml of water to a beagle (n = 6) starved for 12 hours or more. Thereafter, the concentration of Ecadotril metabolite in plasma was measured by using HPLC. The measured value was used as an index for absorbability of Ecadotril.

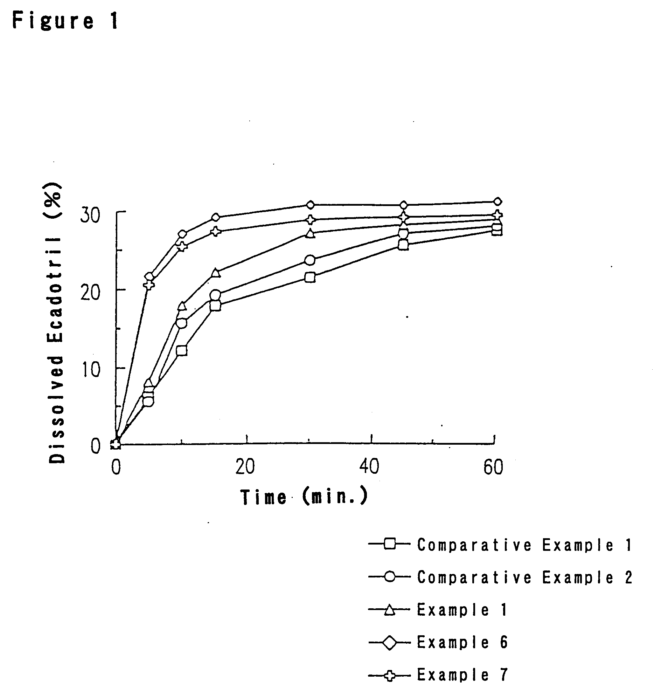

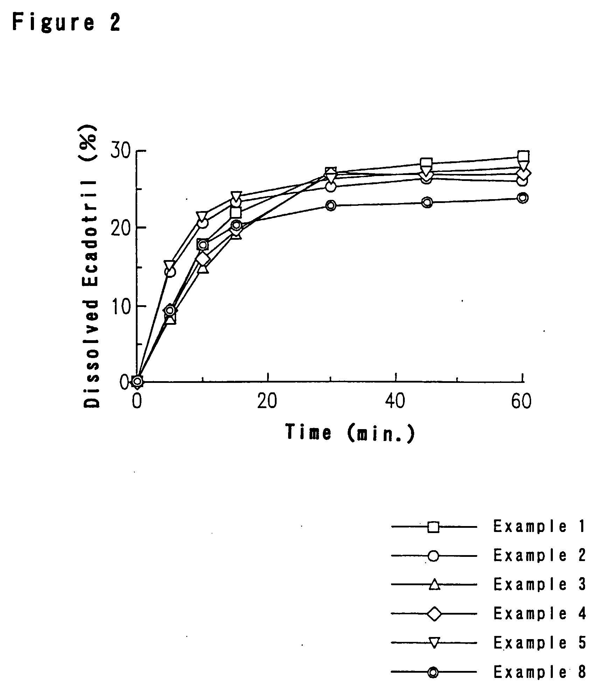

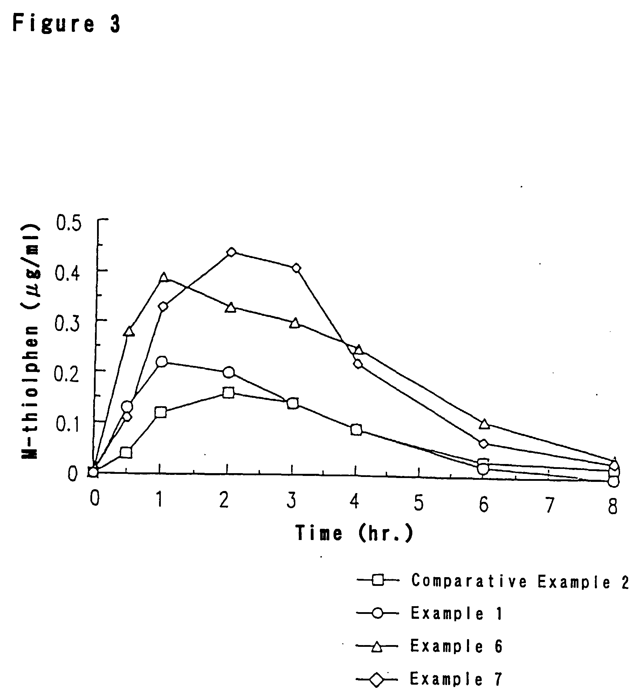

The results of the disintegration test and the dissolution test are shown in Table 2 and Figures **1** and **2**; the results of the absorption test are shown in Figure **3**.

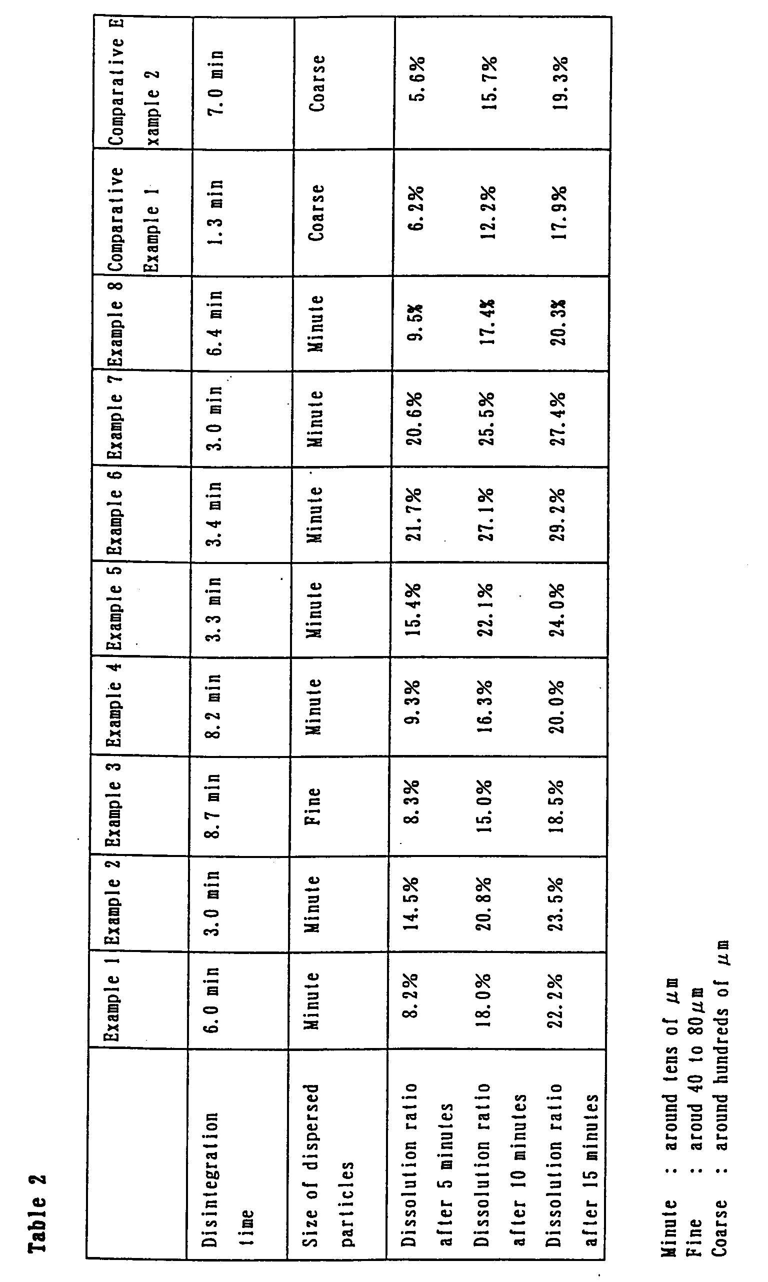

As is apparent from these results, the preparations of the present invention exhibits more excellent dissolution characteristics and absorbability, compared with the preparations which do not contain a wettability improver added thereto. In particular, it is understood that the tablets, produced by mixing the granular preparation of the present invention and the disintegration portions containing a disintegrator (Examples 6 and 7), exhibit excellent dissolution characteristics and absorbability.

### Industrial Applicability of the Invention

According to the present invention, because of the synergistic effect of the improvement of the wettability of Ecadotril and the disintegrator, preparations (granules, powder, tablets, capsules, etc.), in which the rapid-release fine dispersibility of Ecadotril is remarkably enhanced, can be obtained. In particular, the preparation with a high Ecadotril content can be obtained by Spray-Drying method. According to the present invention, Ecadotril is rapidly finely dispersed into primary particles, which are the forms before being subjected to the formulation process, without being formed into larger disintegrated particles. These preparations allow Ecadotril to be rapidly and finely dispersed in the stomach, thereby accelerating the dissolution and absorption of Ecadotril at the area from the duodenum to the upper portion of small intestine, which is rich in bile.

## Claims

1. A method for producing a rapid-release microdispersible preparation containing Ecadotril, comprising the step of treating a mixture containing Ecadotril and a disintegrator with a wettability improver, or the step of combining Ecadotril treated with a wettability improver and a disintegrator.

2. The method of claim 1, wherein the method comprises the steps of preparing an aqueous suspension by wet grinding a mixture containing Ecadotril and the wettability improver, and performing fluidized-bed granulation in which powder containing the disintegrator is sprayed with the aqueous suspension.

3. The method of claim 1, wherein the method comprises the steps of dispersing the disintegrator in an aqueous suspension obtained by wet grinding a mixture containing Ecadotril and the wettability improver to obtain a spray suspension, and performing Spray-Drying in which the spray suspension is spray dried.

4. The method of claim 1, wherein the method comprises the steps of spray drying an aqueous suspension obtained by wet grinding a mixture containing Ecadotril and the wettability improver to obtain fine granules, and adding the disintegrator to the fine granules and mixing the disintegrator with the fine granules.

5. The method of claim 1, wherein the method comprises the steps of wet grinding a mixture containing Ecadotril and the wettability improver to obtain an aqueous suspension, performing coating granulation in which core particles are sprayed with the aqueous suspension, and combining the disintegrator and the particles obtained from the step of coating granulation.

6. The method of claim 1, wherein the wettability improver is a water-soluble polymer or a surfactant.

7. The method of claim 1, wherein the wettability improver is hydroxypropylcellulose.

8. The method of claim 1, wherein the content of the wettability improver in the rapid-release microdispersible preparation is 0.5 to 8 parts by weight based on 100 parts by weight of Ecadotril.

9. The method of claim 1, wherein the disintegrator is sodium crosscarmellose.

10. The method of claim 1, wherein the content of the disintegrator in the rapid-release microdispersible preparation is 5 to 40 parts by weight based on 100 parts by weight of Ecadotril.

11. The method of claim 1, wherein the content of Ecadotril in the rapid-release microdispersible preparation is about 60% by weight or more based on the total weight of the preparation.

12. The method of claim 1, wherein the content of Ecadotril in the rapid-release microdispersible preparation is about 65 to 85% by weight based on the total weight of the preparation.

13. A rapid-release microdispersible preparation containing Ecadotril, wherein the preparation is obtained by the method of any of claims 1 to 12.

14. The preparation of claim 13, which further comprises a disintegration portion.

15. A rapid-release microdispersible tablet containing Ecadotril, wherein the tablet is obtained by tableting the rapid-release microdispersible preparation of claim 13.

16. The tablet of claim 15, wherein the tablet is obtained by the method comprising the steps of dispersing about 15 to 25 parts by weight of disintegrator in the aqueous suspension containing 100 parts by weight of Ecadotril and about 3 to 5 parts by weight of water-soluble polymer to obtain a spray suspension, spray drying the spray suspension to obtain fine granules, and tableting the fine granules, and wherein the content of Ecadotril in the tablet is about 60% by weight or more.

17. The tablet of claim 15, wherein the tablet is obtained by the method comprising the steps of dispersing about 19 to 21 parts by weight of sodium crosscarmellose in the aqueous suspension containing 100 parts by weight of Ecadotril and about 3 to 5 parts by weight of hydroxypropylcellulose to obtain a spray suspension, spray drying the spray suspension to obtain fine granules, and tableting the fine granules, and wherein the content of Ecadotril in the tablet is about 80% by weight.

18. The tablet according to claim 15, wherein the tablet is obtained by the method comprising the steps of dispersing about 19 to 21 parts by weight of sodium crosscarmellose in the aqueous suspension containing 100 parts by weight of Ecadotril with a 50% average particle size of about 10 µm or less and about 3 to 5 parts by weight of hydroxypropylcellulose to obtain a spray suspension, spray drying the spray suspension to obtain fine granules, and tableting the fine granules under a compression pressure of about 0.3 to 0.6 tons, and wherein the content of Ecadotril in the tablet is about 80% by weight.
